# EUROPEAN PATENT SPECIFICATION

(11) **EP 3 489 668 B1**
(45) Date of publication and mention of the grant of the patent: **22.03.2023**
(21) Application number: 19151809.1
(22) Date of filing: 25.11.2015
(51) Int. Cl.: G01N 33/49, G01N 27/07, G01N 27/02

(54) **SYSTEM FOR MIXING AND TESTING A LIQUID**
VORRICHTUNG ZUR MISCHUNG UND ZUM TESTEN EINER FLÜSSIGKEIT
SYSTÈME POUR MÉLANGER ET TESTER UN LIQUIDE

(43) Date of publication of application: 29.05.2019
(62) Divisional of application: 15196214.9
(73) Proprietor: C A Casyso GmbH, 4052 Basel (CH)
(72) Inventor: MADER, Daniel, 79100 Freiburg (DE)
(74) Representative: Müller-Boré & Partner Patentanwälte PartG mbB

(56) References cited:
- WO-A1-99/08107
- WO-A1-2005/059532
- WO-A1-2005/107939
- JP-A- 2001 188 070
- US-A- 5 491 408
- US-A1- 2001 024 803
- US-A1- 2002 137 234
- US-A1- 2006 024 206
- US-A1- 2009 261 803
- MORONEY R M ET AL: "Fluid motion produced by ultrasonic Lamb waves", 19901204; 19901204 - 19901207, 4 December 1990 (1990-12-04), pages 355-358, XP010010171,

## Description

The present invention relates to a system for mixing and testing a liquid, in particular for mixing a liquid containing blood platelets in order to perform platelet impedance aggregometry (PIA).

### Related art

Platelet impedance aggregometry systems and methods are used for the measurement of a thrombocyte function and drug-related impact on full blood coagulation. Conventionally, a mixer device is used for each measurement cell in order to mix reagents with the blood and to induce mechanical stress in the blood cells by the resulting shear forces in the moving liquid.

US 8 877 510 B2 describes a method for conducting platelet aggregation analysis using a cartridge device having a receiving portion for receiving a blood sample and a jack portion for receiving a plug. Prior to and during measurement, a magnetic stir bar is activated by an electromagnet placed under the receiving portion. Conventional magnetic stir bar systems always have to be formed with certain minimal dimensions in order for the stir bar mechanism to work because the stir bar needs a minimum mass and cross section for robust contactless magnetic actuation and effective liquid displacement.

WO 2005/059532 A1 describes a cartridge device comprising a receiving portion for receiving a blood sample and a jack portion for receiving a plug; a stirring device for circulating said blood sample within said receiving portion; and an electrode holder having at least one incorporated electrode wire pair; wherein the electrode holder is attachable to the cell such that one end of the at least one electrode wire pair forms a sensor unit for measuring the electrical impedance between the two electrode wires of the at least one electrode wire pair within the blood sample and that the opposite end of the at least one electrode wire pair forms a plug portion being connectable directly to the plug for an electrical connection of the sensor unit to an analyser.

WO 2005/107939 A1 describes a complicated operation or process which is performed by generating acoustic waves or oscillation by a piezoelectric device. Equipment includes the piezoelectric device provided with a piezoelectric element composed of a piezoelectric material, and a plurality of electrodes, which are arranged on the both planes of the piezoelectric element and are formed by a metal thin film pattern. On the piezoelectric element, a groove is arranged at a position on the external side of the electrodes.

US 2009/261803 A1 describes an aggregometer for measuring and recording platelet aggregation with at least one measurement cuvette having two electrodes, with a device for moving the liquid to be examined and with measurement and evaluation electronics connected to the electrodes via contact elements, wherein the measurement cuvette comprises two adjacently arranged, upwardly open partial spaces which are only connected to one another in the lower region, in that the electrodes are designed as metal spheres inserted in the bottom of the measurement cuvette and in that the device for moving the liquid is a pumping device which can be placed on one of the partial spaces in a substantially sealing manner and which periodically and alternately generates low and excess pressure. WO 99/08107 A1 describes an aggregometer which is economical and accurate. The aggregometer includes a disposable test cell having two electrode tips. Adjacent parallel planar surfaces of the electrode tips define a channel which is substantially parallel to a flow direction of the sample prior to entering the channel. Also provided is a method for analyzing platelet aggregation by measuring electrical impedance in a sample using the aggregometer.

US 2002/137234 A1 describes an assay apparatus which includes a cell with a working electrode and a sonicating device structurally coupled to the cell for sonication of the contents of the cell.

There is a need for an efficient and reliable system for mixing and testing a liquid wherein the receiving portion for receiving the liquid is highly miniaturizable.

This problem is solved by the independent claim. Particular embodiments are defined in the dependent claims.

### Disclosure of the invention

The invention provides, according to a first aspect, a system for mixing and testing a liquid according to independent claim 1.

The liquid may in particular be a liquid containing blood platelets. For example, the liquid may comprise full blood, reagents and/or saline solution. The reagents may in particular comprise, or consist of, reagents affecting blood coagulation.

The ultrasound waves may in particular be so-called surface acoustic waves (SAW). Lamb waves propagate in solid plates or plane sections of solids; they were first introduced in the scientific publication "On Waves in an Elastic Plate", Lamb, H., Proceedings of the Royal Society of London, Series A, Containing Papers of a Mathematical and Physical Character (1905-1934), 1917-03-01.93 (648):114-128. Lamb waves are elastic waves where the particle motion lies in a plane that contains the direction of wave propagation and the plate normal, i.e. the direction perpendicular to the plate. Lamb waves may have symmetric modes, for example extensional modes and/or asymmetric modes, for example flexural modes.

The electrical property of the fluid to be determined may in particular be an electrical impedance, i.e. a measure of the opposition that the fluid presents to an alternating current when the electrical voltage is applied between the first electrode and the second electrode. As an alternative, the electrical property to be determined may be an electrical resistance. The first and a second electrode are configured so as not to touch each other inside the receiving portion, accordingly. Any or all of the elements of the system, as long as practical, may be realized as hardware, as software or as a combination of both hardware and software. Elements handling electrical signals and currents may be realized in analog technologies, in digital technologies or in a combination of both analog and digital technologies.

### Advantages of the invention

An underlying idea of the invention is that Lamb waves can be created in at least a section of the receiving portion using ultrasound transducers. The Lamb waves in the receiving portion translate, via a mode conversion effect, into a motion of the liquid inside the receiving portion and thus lead to a mixing of the fluid within the receiving portion. In particular, the liquid in the receiving portion, which forms a measurement cell, can be moved in a controlled and continuous manner without the need of a mechanical stir bar.

Lamb waves are well suited for mixing a liquid containing blood platelets for subsequent analysis. In particular, Lamb waves provide an advantageously homogeneous coupling of kinetic energy into the liquid, avoiding large energy density or momentum spikes that could potentially damage the blood platelets.

The receiving portion and the mixing unit, which conventionally must have a certain minimum size, are, according to the present invention, well suited for a miniaturization and automation and systems with cheap consumables and low sample volume consumption. Conventionally, either stir bars had to be added to a disposable cell or had to be part of the disposable cell, increasing the required effort and cost in both cases.

Further advantageously, according to the present invention, no part of the mixing unit has to be in direct contact with the liquid, and, in particular, no such part has to be extracted again from the mixing unit. Especially when the liquid comprises full blood or blood platelets, hygiene and/or medical safety criteria may be more easily met by the system and method of the present invention. Additionally, reagents may be pre-positioned inside the receiving portion, e.g. inside a cartridge.

Because of the relatively few constraints in the design of the mixing unit in particular, by selecting appropriate materials and signals, almost arbitrary frequencies and amplitudes can be generated and combined in the liquid. Thus, the non-homogenous mechanical force field desired for platelet impedance aggregometry can be tailored towards sample volume and measurement cell dimensions. The present invention is thus usable in a large variety of cases.

The omission of separate mixing and measuring/reagent chambers allows for a simplified design of a measurement disposable, e.g. a cartridge, and the system itself so that system reliability can be increased at decreasing costs.

Further advantageous embodiments of the present invention may be found in the dependent claims as well as in the description in combination with the Figures.

According to an embodiment of the system according to the first aspect of the invention, the system comprises a cartridge and a testing apparatus. The receiving portion and the first and the second electrodes are integrated into the cartridge. The cartridge is adapted to be brought into engagement with the testing apparatus such that the mixing unit is positioned to emit the ultrasound waves directed towards the receiving portion when the cartridge is brought into engagement with the testing apparatus.

The cartridge may be brought into engagement with the testing apparatus e.g. by inserting the cartridge into the testing apparatus, by plugging the cartridge into the testing apparatus and so on. This allows for a creation of a relatively simple disposable cartridge with one or more measurement cells, whereas more intricate and/or more expensive elements of the system are comprised in the re-usable testing apparatus. Thus, system reliability may be further increased at decreasing costs.

According to another example, not according to the present invention, the cartridge comprises a first electrical interface and the testing apparatus comprises a second electrical interface. The voltage application unit is integrated into the testing apparatus and is adapted to apply the electrical voltage to the first and the second electrodes over the first and the second electrical interfaces when the cartridge is brought into engagement with the testing apparatus, in which state the first and the second electrical interfaces are electrically coupled, i.e. connected.

Electrical power may be provided to the voltage application unit by a power source of the testing apparatus, for example a connection to the general power grid or a rechargeable or non-rechargeable battery of the testing apparatus. In this way, system reliability may be further increased at decreasing costs.

According to another embodiment of the first aspect of the invention, the cartridge comprises a first electrical interface and the testing apparatus comprises a second electrical interface and the measuring unit is integrated into the testing apparatus. The measuring unit is electrically coupled to the first and the second electrodes over the first and the second electrical interface when the cartridge is brought into engagement with the testing apparatus.

The electrical interfaces used for coupling the measuring unit to the first and second electrode may also be used for applying the electrical voltage of the voltage application unit to the first and second electrical interface as described above. Thus, a technically demanding measuring unit can be provided once in a testing apparatus and can be used for a plurality of cartridges, increasing system reliability at decreasing costs.

According to another embodiment of the first aspect of the present invention the mixing unit comprises at least one piezo-electric element and a frequency generator adapted to generate a periodic electrical signal to be applied to the at least one piezo-electric element so that the at least one piezo-electric element emits the ultrasound waves towards the receiving portion. The frequency of the periodic electrical signal may be identical to a resonance frequency of the piezo-electric element. As an alternative, any other ultrasound transducer may be provided instead of the piezo-electric element. The ultrasound waves may be emitted with a frequency of 10 MHz or less, preferably 2 MHz or less, in particular 1 MHz or less, especially 0.5 MHz or less.

One or more piezo-electric elements and/or ultrasound transducers may be provided for each receiving portion comprised in the system. The piezo-electric elements may be provided with a dimension, or dimensions, similar to or larger than a dimension, or dimensions, of the receiving portion. For example, a cross section of the piezo-electric element in a virtual plane may be equal to, or larger than, a cross-section of the receiving portion in the same virtual plane, in particular a virtual plane perpendicular to a longitudinal axis of the piezo-electric element. A diameter of the receiving portion contained in a virtual plane may be equal to, or smaller than, a width of the piezo-electric element within the same virtual plane.

According to another embodiment of the first aspect of the present invention, the frequency generator is adapted to generate the periodic electrical signal, and apply the periodic electrical signal to the at least one piezo-electric element, with a frequency of 10 MHz or less, preferably 2 MHz or less, in particular 1 MHz or less, especially between 0.5 MHz and 1 MHz. These frequencies may allow the use of technically less demanding configurations of the frequency generator. In addition, these frequency ranges are especially well suited for inducing Lamb waves in the receiving portion or in a section of the receiving portion. A piezo-electric element of the mixing unit may be chosen such that its resonance frequency or one of its resonance frequencies lies within these frequency ranges and, in particular, coincides with the frequency of the generated periodic electrical signal and/or with the frequency of the Lamb waves to be induced in at least the section of the receiving portion.

According to the present invention the system comprises a cartridge and a testing apparatus, wherein the receiving portion, the first and the second electrodes, the voltage application unit and the at least one piezo-electric element are integrated into the cartridge and wherein the frequency generator is integrated into the testing apparatus. The cartridge is adapted to be brought into engagement with the testing apparatus such that the periodic electrical signal generated by the frequency generator is applied to the at least one piezo-electric element and such that the at least one piezo-electric element is positioned to emit the ultrasound waves towards the receiving portion when the periodic electrical signal is applied to the piezo-electric element. In this way, the piezo-electric element may be made a part of the disposable cartridge, allowing a more precise positioning of the piezo-electric element with respect to the receiving portion and thus allowing a more precise control of the emitted ultrasound waves.

According to the present invention, the receiving portion is formed as a double-open-ended tube structure. The tube structure can be, for example, a microchannel. A mixing unit may be adapted to emit the ultrasound waves towards a wall section of the tube structure. The mixing unit may comprise a piezo-electric element strip with a longitudinal axis of the strip parallel to an axial direction of the tube structure. The first and/or the second electrode may be introduced into the tube structure or guided through the tube structure in the axial direction of the tube structure. A capillary effect may be exploited in order to introduce the liquid into the receiving portion.

According to still another embodiment of the first aspect of the present invention, the voltage application unit is adapted to apply an alternating voltage between the first electrode and the second electrode. The measuring unit may be adapted to determine an impedance of the fluid in response to the applied alternating voltage between the first and the second electrode. In other words, the measuring unit is adapted to measure the impedance offered by the fluid against the applied alternating voltage. In this way, platelet impedance aggregometry may be performed.

According to another embodiment of the first aspect of the present invention, the receiving portion is adapted to hold a maximum volume of 250 mm³ or less, for example 100 mm³ or less, preferably 75 mm³ or less, in particular 60 mm³ or less. Thus, a cartridge containing the receiving portion may be further miniaturized and/or more receiving portions may be provided at a single cartridge of constant size, reducing effort for testing a large number of samples, e.g. blood samples. In addition, the necessary sample volume of the liquid for the testing may be reduced: conventionally large diameters of receiving portions were necessary to accommodate the magnetic stir bars such that a large volume of the liquid to be tested was necessary to fill the receiving portion up to a desired level.

According to an embodiment of the present invention, the emitting of the ultrasound waves comprises generating a periodic electrical signal with a frequency of 10 MHz or less, preferably 2 MHz or less, in particular 1 MHz or less and applying the generated periodic electrical signal to at least one piezo-electric element. For example, the generated periodic electrical signal may be applied to a piezo-electric element as described with respect to the first aspect of the present invention.

According to further embodiments of the present invention, an alternating voltage is applied to the first electrode and the second electrode and an impedance of the fluid is determined in response to the applied alternating voltage.

In further embodiments of the present invention, the impedance of the fluid in response to the applied alternating voltage is continuously measured over a predetermined measuring time, preferably a measuring time longer than one minute, in particular longer than five minutes.

According to further embodiments of the present invention, the liquid comprises, or consists of, a first liquid component comprising blood platelets, e.g. full blood and a second liquid component, e.g. saline solution and/or reagents. Preferably, the emitting of the ultrasound waves is performed for a predetermined emitting time in order to mix the first liquid component and the second liquid component. Advantageously, the measuring time overlaps the emitting time partially or completely.

The embodiments, modifications and variants described above and in the following may be freely combined with one another as long as they fall within the scope of the appended claims. Further possible embodiments, modifications and realizations of the invention may also include not explicitly described combinations of the features described in the foregoing and in the following with respect to certain embodiments. In particular, the skilled person will be able to add single features of some embodiments to other embodiments to improve their function, within the scope of the claims.

### Brief description of the drawings

The above and other objects, features and other advantages of the present invention will be more clearly understood from the following detailed description taken in conjunction with the accompanying drawings, in which (the exemplary systems are not according to the present invention):
Fig. 1 shows a block diagram of an exemplary system for mixing and testing a liquid;
Fig. 2 shows an exemplary system for mixing and testing a liquid;
Fig. 3 shows an exemplary system for mixing and testing a liquid;
Fig. 4A - 4D show schematic views of a possible assembly of certain elements of the system;
Fig. 5A - 5D show schematic views of another possible assembly of certain elements of the system;
Fig. 6 shows a schematic cross section of yet another possible assembly of certain elements of the system;
Fig. 7 shows a schematic flow diagram illustrating a method for mixing and testing a liquid (not according to the present invention); and
Fig. 8 shows a schematic flow diagram illustrating a method for mixing and testing a liquid (not according to the present invention).

The figures are intended to further understanding of the embodiments of the invention. The figures illustrate embodiments and serve, together with the following detailed description, to explain concepts and principles of the invention. The elements of the figures are not necessarily drawn true to scale. The same reference signs are intended to designate the same or similar elements throughout the drawings if nothing to the contrary is explicitly indicated.

Although the terms such as first, second, etc. may be used herein to describe various elements, these elements should not be interpreted as limited by these terms. These terms are used to distinguish one element from another. For example, a first element could be termed a second element, and, similarly, a second element could be termed a first element, without departing from the scope of the claims.

### Detailed description of the drawings

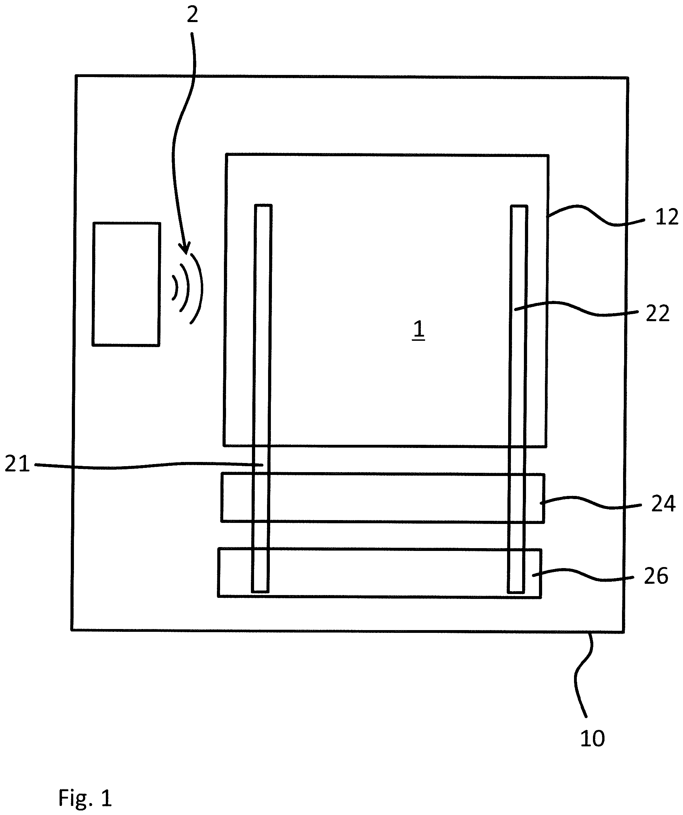

Fig. 1 shows a block diagram of a system 10 for mixing and testing a liquid 1. The system 10 comprises a receiving portion 12 for receiving the liquid 1 to be tested and a mixing unit 18 adapted to emit ultrasound waves 2, in particular with a frequency of 10 MHz or less, directed towards the receiving portion 12. The mixing unit 18 is adapted to emit the ultrasound waves 2 in such a way that the ultrasound waves 2 excite Lamb waves in at least a section 14 of the receiving portion 12, in particular a wall section 14 or a bottom section of the receiving portion 12.

The system 10 further comprises at least a first electrode 21 reaching into the receiving portion 12 and a second electrode 22 reaching into the receiving portion 12, i.e. into a volume within the receiving portion 12 that is intended to contain the liquid 1 for mixing and testing. The first and the second electrodes 21, 22 are e.g. formed of platinum. The electrodes 21, 22 may, for example, be configured as thin rods with a diameter of 250 micrometers or less and/or a length of 3 millimeters each. The electrodes 21, 22 may be positioned 250 micrometers apart from each other in parallel to each other.

The system 10 further comprises a voltage application unit 24 adapted to apply an electrical voltage between the first electrode 21 and the second electrode 22 and a measuring unit 26 adapted to determine an electrical property of the fluid 1 in response to the applied electrical voltage. In other words, the voltage application unit 24 is adapted to create a difference in electrical potential between the first and the second electrode 21, 22. In particular, the determined electrical property may be a resistance or an impedance offered by the fluid 1 to a direct or alternating current between the first and the second electrode 21, 22 through the liquid 1 based on, and/or induced by, the applied electrical voltage.

The voltage application unit 24 as well as the measuring unit 26 are electrically coupled, or configured to be able to be coupled, to the first and the second electrode 21, 22, for example over electrical interfaces.

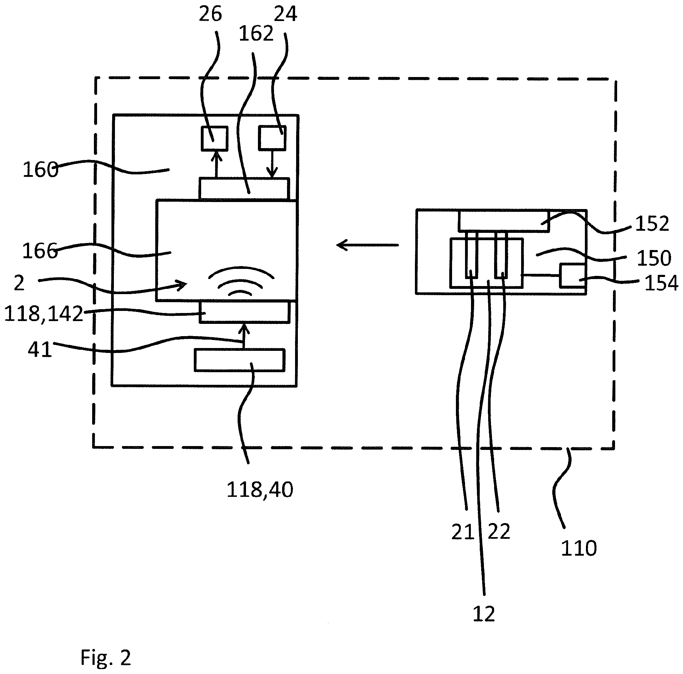

Fig. 2 depicts a system 110 for mixing and testing a liquid 1. The system 110 is a variant of the system 10 and comprises, preferably consists of, a cartridge 150 and a testing apparatus 160. The receiving portion 12 with the first and the second electrode 21, 22 reaching into the receiving portion 12 are integrated into the cartridge 150. The cartridge 150 is configured with an inlet 154 which is adapted to receive at least one liquid 1 from outside the cartridge 150 and to conduct the received at least one liquid 1 into the receiving portion 12 150. Two or more receiving portions 12 may be provided in a single cartridge 150. Each receiving portion 12 may be provided with its own inlet 154. Alternatively, some or all of the receiving portions 12 may be provided with the liquid 1 to be tested by a single inlet 154 of the cartridge 150. One or more piezo-electric elements may be provided for each receiving portion provided in the cartridge.

The testing apparatus 160 comprises a docking portion 166 adapted to receive the cartridge 150. The mixing unit 118 is integrated into the testing apparatus 160. The cartridge 150 is adapted to be brought into engagement with the testing apparatus 160, in particular with the docking portion 166 of the testing apparatus 160 such that the mixing unit 118 is positioned to emit the ultrasound waves 2 directed 20 towards the receiving portion 12, or towards all of the receiving portions 12 of the cartridge 150, if there is more than one.

The mixing unit 118 comprises a frequency generator 40 and at least one piezoelectric element 142, both integrated into the testing apparatus. The at least one piezo-electric element 142 is preferably positioned as part of, or adjacent to, a section of the docking portion 166 that is adjacent to the cartridge 150, in particular the receiving portion 12 or all of the receiving portions 12 of the cartridge 150 when the cartridge is brought into engagement with the docking portion 166. The mixing unit 118 may comprise a coupling element configured to transport the emitted ultrasound wave from the piezo-electric element 142 to the docking portion 166 and/or to the cartridge 150.

The docking portion 166 may be configured as a docking bay or a slot or the like in the testing apparatus 160.

The frequency generator 40 is adapted to generate a periodic electrical signal 41, for example a sine wave, in particular with a frequency of 10 MHz or less. The frequency generator 40 is further adapted to apply the generated periodic electric signal 41 to the at least one piezo-electric element 142. The piezo-electric element 142 is adapted to emit, when the periodic electrical signal 41 is applied to the piezoelectric element 142, the ultrasound waves towards the receiving portion 12 when the cartridge 150 is brought into engagement with the docking portion 166. The piezo-electric element 142, or any of the other piezo-electric elements described herein, may contain, or consist of, naturally occurring crystals, synthetic crystals like gallium orthophosphate or Langasite or synthetic ceramics. Exemplary synthetic ceramics for the piezo-electric element include, but are not limited to, barium titanate, lead zirconate titanate, commonly known as PZT, potassium niobate, lithium niobate, lithium tantalate, sodium tungstate and zinc oxide.

The cartridge 150 is configured with a first electrical interface 152 which is separately coupled to the first and the second electrodes 21, 22 such as to be able to apply a voltage to the first and the second electrodes 21, 22 and such that the electrical property of the liquid 1 may be determined in response to the applied electrical voltage. The testing apparatus 160 is provided with a second electrical interface 162. The cartridge 150, the first electrical interface 152, the testing apparatus 160, the second electrical interface 162 and the docking portion 166 are configured such that the first and the second electrical interfaces 152, 162 are brought into electrical connection with each other when the cartridge 150 is brought into engagement with the docking portion 166.

The voltage application unit 24 and the measuring unit 26, e.g. as described above with respect to the system 10, are integrated into the testing apparatus 160 and are adapted to perform their functions over the electrical connection provided by the first and the second electrical interfaces 152, 162 when they are arranged in electrical connection with each other.

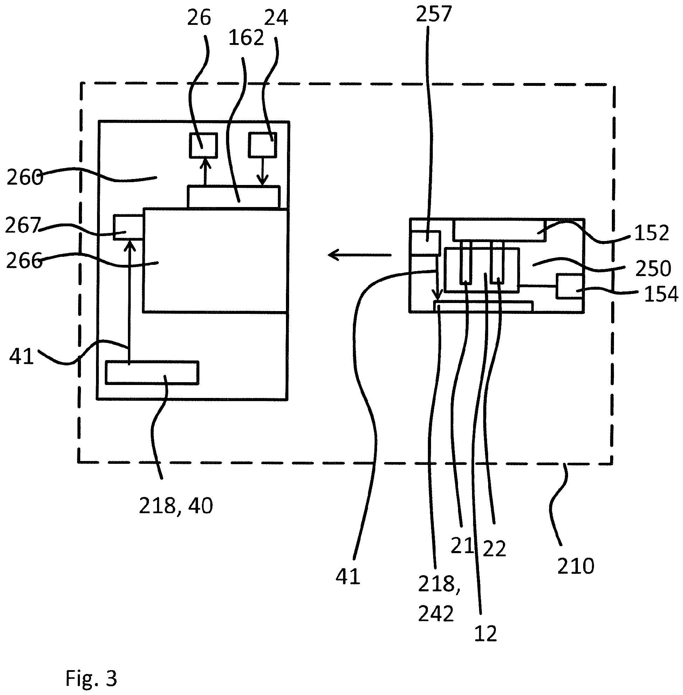

Fig. 3 shows a system 210 for mixing and testing a liquid 1. The system 210 is a variant 35 of the system 110 and differs from the system 110 in the configuration of a mixing unit 218 of the system 210 and of corresponding electrical connections in an apparatus 260 and a cartridge 250 of the system 210. All of the elements of the system 210, or features thereof, not explicitly described as different from the system 10 and/or 110 may be configured in the same way as described for the system 10 and/or 110 or in the foregoing.

In the system 210, the mixing unit 218 comprises a frequency generator 40 integrated into the testing apparatus 260 as described for the system 110 and the testing apparatus 160. The mixing unit 218 further comprises a piezo-electric element 242 integrated into the cartridge 250 as described for the piezo-electric element 142 and the cartridge 150.

The cartridge 250 is further provided with a third electrical interface 257. A docking portion 266 of the testing apparatus 260 is provided with a fourth electrical interface 267. The third and the fourth electrical interfaces 257, 267 and the cartridge 250 and the docking portion 266 are configured such that the third and the fourth electrical interfaces 257, 267 are brought into electrical contact with each other when the cartridge 250 is brought into engagement with the docking portion 266 of the testing apparatus 260. Signal connections are provided within the testing apparatus 260 and the cartridge 250 such that the periodic electrical signal 41 generated by the frequency generator 40 can be applied to a piezo-electric element 242 over the third and fourth electrical interfaces 257, 267, when the cartridge 150 is brought into engagement with the docking portion 166.

The piezo-electric element 242 may be configured in the same way as described above with respect to the piezo-electric element 142.

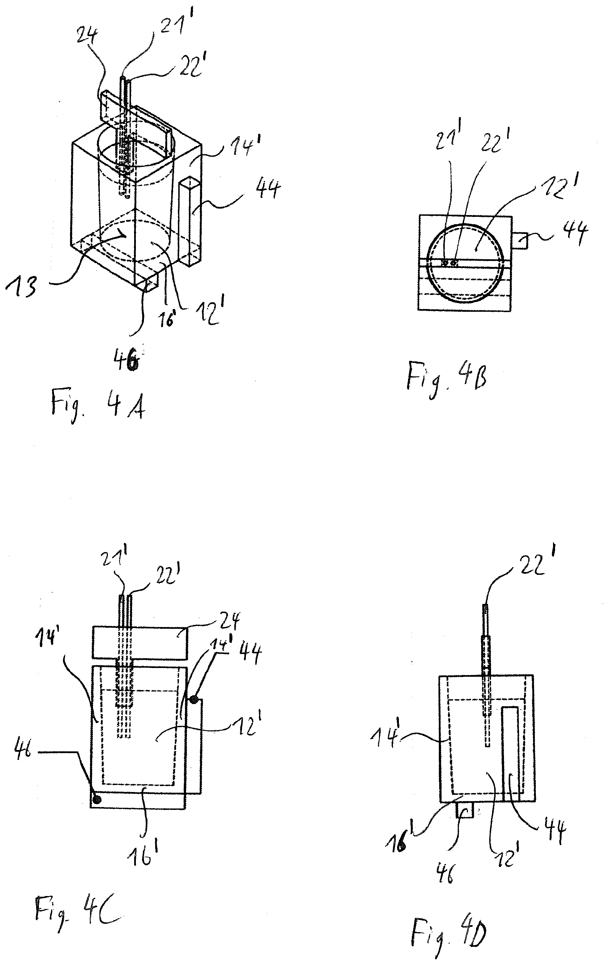

Figures 4A to 4D depict schematic views of a possible assembly of certain elements of the system, in particular to one of the systems 10, 110 and/or 210 as described above.

Fig. 4A shows a diagonal top/front/side view, Fig. 4B a top view, Fig. 4C a front view and Fig. 4D a side view of the same assembly of a receiving portion 12', a first and a second electrode 21', 22' a printed circuit board 24 and two piezo-electric elements 44, 46. The receiving portion 12' is formed by four wall portions 14' and a bottom portion 16' into which the first and the second electrode 21', 22' reach which are connected to a printed circuit board, PCB, 24. The PCB may connect the first and the second electrodes 21', 22' to the voltage application unit 24 and/or to the measuring unit 26. Figure 4A to Figure 4D show the assembly of the two piezo-electric elements 44, 46 in close proximity to the receiving portion 12'. The piezo-electric elements 44, 46 may be provided together or as alternatives to each other.

A first piezo-electric element 44 is provided as a strip or cuboid, e.g. consisting of or comprising PZT, with a longitudinal axis of the cuboid parallel to an axial direction of the receiving portion 12' and/or perpendicular to a bottom surface 13 formed by the bottom portion 16' inside the receiving portion 12'. A second piezo-electric element 46 is provided as a strip or cuboid, e.g. consisting of or comprising PZT, with a longitudinal axis of the cuboid or strip arranged perpendicular to a normal vector of the surface 13.

The receiving portion 12' is provided in the form of a hollow cylinder or of a hollow truncated cone, wherein the axial direction of the receiving portion 12' is given by a rotational symmetry axis of the receiving portion 12'. The first piezo-electric element 44 and the second piezo-electric element 46 are positioned distanced from said rotational symmetry axis.

The receiving portion 12' may be configured with a diameter of the hollow cylinder of 8 millimeters or less, preferably five millimeters or less, in particular four millimeters or less. The receiving portion may be adapted to hold a maximum volume of 250 mm³ or less, for example 100 mm³ or less, preferably 75 mm³ or less, in particular 60 mm³ or less. The electrodes 21', 22' may be positioned such that there is a distance of 5 millimeters or less between the bottom portion 16' of the receiving portion 12' and the electrodes 21', 22', in particular 3 millimeters or less. The electrodes 21', 22' may be positioned distanced from each other in a radial and/or transverse direction with respect to the cylinder. Alternatively, the electrodes 21', 22' may be distanced from each other in an axial direction. The electrodes may be provided with a tilt with respect to the rotational symmetry axis or in parallel to the rotational symmetry axis.

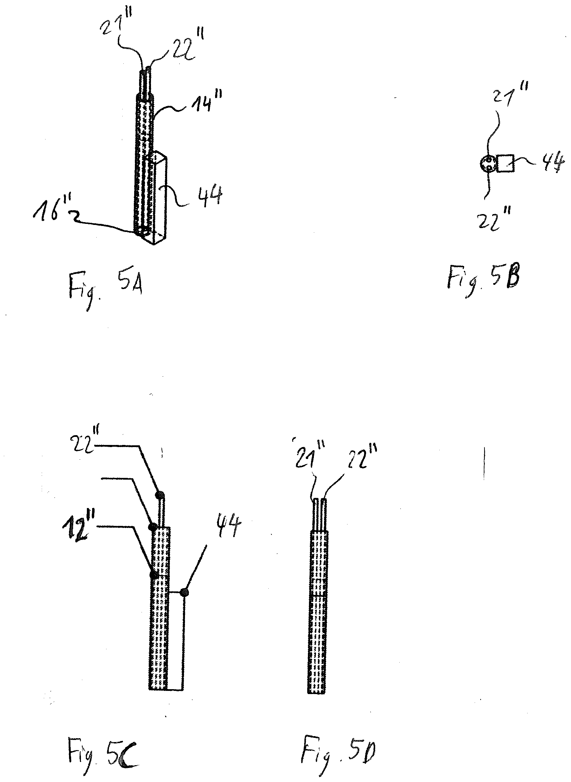

Figures 5A to 5D depict schematic views of another possible arrangement of certain elements of the system, in particular to one of the systems 10, 110 and/or 210 as described above.

Fig. 5A shows a diagonal top/front/side view, Fig. 5B a top view, Fig. 5C a front view and Fig. 5D a side view of the same assembly of a receiving portion 12", a first and a second electrode 21", 22" and a piezo-electric element 44. The receiving portion 12" is formed by a cylindrical wall portion 14" and a bottom portion 16" into which the first and the second electrode 21", 22" reach. Figure 5A to Figure 5D show the assembly of the piezo-electric element 44, as described above with respect to Figures 4A to 4D and the receiving portion 12', in close proximity to the receiving portion 12".

The piezo-electric element 44 is provided as a strip or cuboid, e.g. consisting of or comprising PZT, with a longitudinal axis of the cuboid parallel to an axial direction of the receiving portion 12‴.

The receiving portion 12" is provided in the form of a hollow cylinder, wherein the axial direction of the receiving portion 12" is given by a rotational symmetry axis of the receiving portion 12". The piezo-electric element 44 is positioned distanced from said rotational symmetry axis. A cross-section of the receiving portion 12", in particular of the volume intended to be occupied by the liquid 1, in a plane perpendicular to the rotational symmetry axis of the receiving portion 12" is smaller than a cross-section of the piezo-electric element 44 in the same plane. A diameter of the cylinder of the receiving portion 12" is equal to a width of the piezo-electric element 44. A length of the piezo-electric element 44 is smaller than an axial length of the cylinder of the receiving portion 12".

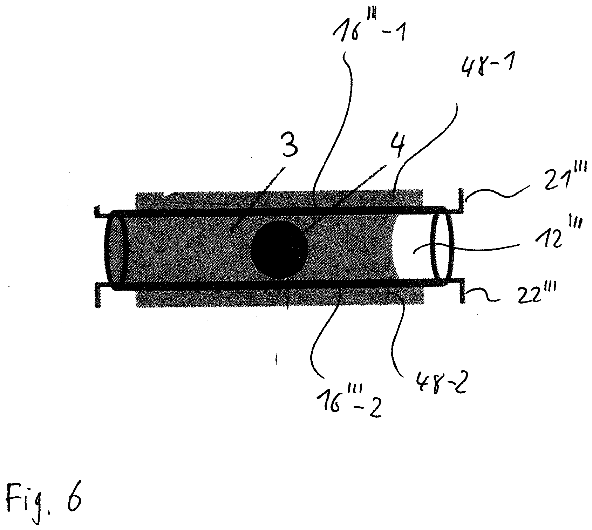

Fig. 6 shows the assembly of first and second electrodes 21‴, 22‴ and a receiving portion 12‴ as used in the system according to the present invention, in particular with the systems 10, 110, 210 as described above.

According to Fig. 6, the receiving portion 12‴ is formed as a double-open-ended tube structure through which the first and the second electrodes 21‴. 22‴ are guided, entering the tube structure on one end and exiting the tube structure on the opposite end, the first and the second electrode 21‴, 22‴ being provided as wires at, e.g. diametrically opposite, interior sides of the tube structure of the receiving portion 12". The tube structure 12‴ may be formed of plastics, for example of a two-part molded plastic.

In Fig. 6, schematically full blood 3 as a first liquid component and reagents 4 as a second liquid component are depicted as component parts of the liquid 1 that are to be mixed into each other, in particular prior to and/or simultaneous with applying the electrical voltage between the first and the second electrode 21‴, 22". A third liquid component, e.g. saline solution and/or additional liquid components may be added to the fluid 1 or as part of the fluid 1 and may be mixed prior to and/or simultaneous with applying the electrical voltage.

In Fig. 6, a first piezo-electric element 48-1 and a second piezo-electric element 48-2 are depicted arranged at diametrically opposite sides of the tube structure of the receiving portion 12‴ outside the receiving portion 12". The first and the second piezo-electric elements 48-1, 48-2 may be provided together or as alternatives to each other. The first piezo-electric element 48-1 and/or the second piezo-electric element 48-2 may be provided integrated into a cartridge, e.g. the cartridge 150 or 250, comprising the receiving portion 12‴ or may be integrated into a testing apparatus, e.g. the testing apparatus 160 or 260, separate from the cartridge comprising the receiving portion 12‴. Alternatively, the first piezo-electric element 48-1 may be integrated into the testing apparatus and the second piezo-electric element 48-2 into the cartridge or vice versa.

Regarding the system 10, 110, 210, the first and second electrodes 21, 22 may be provided as described for the first and second electrodes 21', 22'; 21", 22"; 21", 22", the piezo-electric elements 142; 242 may be provided as described for the piezoelectric elements 44, 46, 48-1, 48-2 and/or the receiving portion 12 may be provided as described for the receiving portion 12'; 12"; 12", as described with respect to Figures 4A to 6.

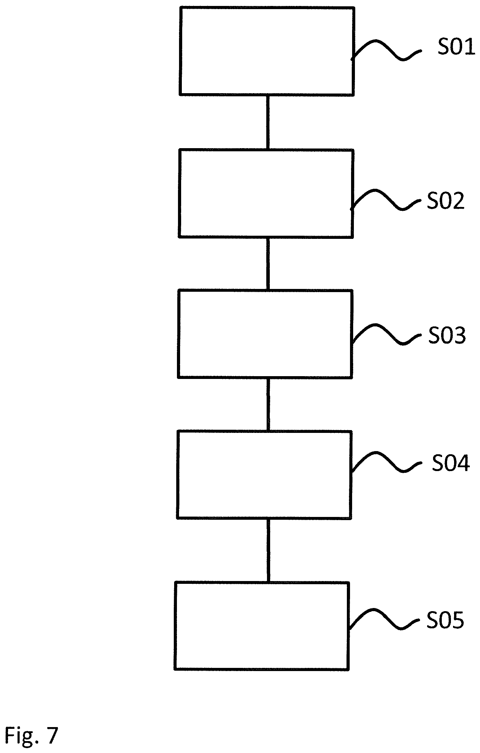

Fig. 7 shows a schematic flow diagram illustrating a method for mixing and testing a liquid 1. This method may be performed with the system according to embodiments of the present invention, in particular with the systems 10; 110; 210 and/or the arrangement variations depicted in Figures 3A-5. The method may be adapted according to any, multiple, or all advantageous modifications and variations of the above embodiments of the system and vice versa.

In a first step S01, the liquid 1 to be tested is provided in a receiving portion 12; 12'; 12"; 12‴. In a step S02 ultrasound waves 2 are emitted towards the receiving portion 12; 12'; 12"; 12‴. This is done in order to excite Lamb waves in at least a section 14; 14', 16'; 14", 16"; 16"'-1, 16"'-2 of the receiving portion 12; 12'; 12". The emitting S02 is performed, at least also, after the liquid 1 is provided in the receiving portion 12. Because of the mode conversion effect, the excited Lamb waves in at least the section 14; 14', 16'; 14", 16"; 16"'-1, 16"'-2 of the receiving portion 12; 12'; 12"; 12‴ have the effect of mixing the liquid 1 by inducing mechanical movement and shear forces inside the liquid 1. The liquid 1 may comprise, or consist of, a first liquid component 3, in particular full blood or containing blood platelets and a second liquid component 4, in particular saline solution and/or reagents. The reagents preferably are reagents affecting blood coagulation.

In a step S03, a first electrode 21; 21'; 21"; 21‴ and a second electrode 22; 22'; 22"; 22‴ are brought into contact with the liquid 1 in the receiving portion 12; 12'; 12"; 12'". The first and the second electrode 21; 21'; 21"; 21‴, 22; 22'; 22"; 22‴ may be fixedly arranged with respect to the receiving portion 12; 12'; 12"; 12‴ such that the providing S03 of the contact between the first electrode and the second electrode 21, 22; 21', 22'; 21", 22"; 22", 22‴ and the liquid 1 is performed by way of the providing S01 of the liquid 1 in the receiving portion 12; 12'; 12"; 12‴, for example after the liquid 1 has achieved a certain liquid level inside the receiving portion 12; 12'; 12"; 12‴. This liquid level at which the contact is formed may e.g. be five millimeters or less, preferably three millimeters or less, from the bottom portion 16' of the receiving portion 12'.

In a step S04, an electrical voltage is applied between the first electrode 21; 21'; 21"; 212‴ and the second electrode 22; 22'; 22"; 22‴ in contact with the liquid 1. Preferably, an alternating voltage is applied. In a step S05, an electrical property of the fluid 1 in a receiving portion 12; 12'; 12"; 12‴ is measured in response to the applied electrical voltage. In particular, an impedance of the fluid 1 is measured in response to the applied alternating electrical voltage.

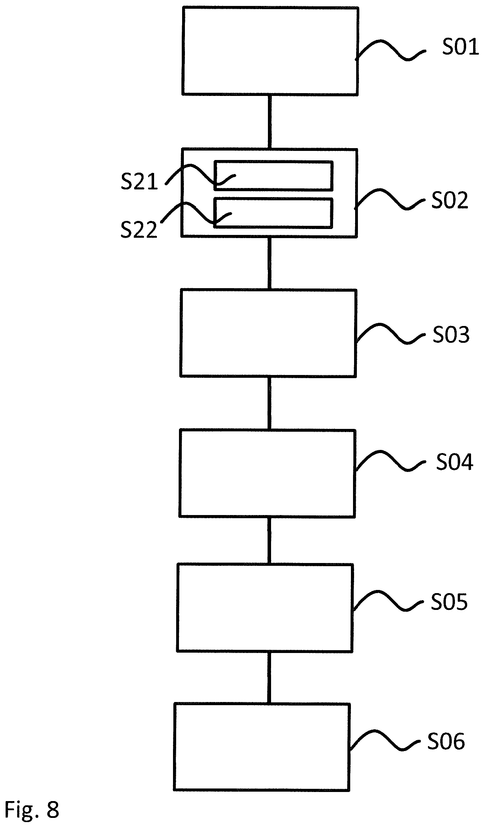

Fig. 8 shows a schematic flow diagram of a method for mixing and testing a liquid 1 which is a variant of the method described with respect to Fig. 6. The method of Fig. 7 differs from the method of Fig. 6 in that, in particular, the step of emitting S02 the ultrasound waves comprises a first sub-step S21 and a second sub-step S22. in the first sub-step S21, a periodic electrical signal 41 with a frequency of 10 MHz or less is generated, for example with the frequency generator 40 of the system 110, 210 as described above. In the second sub-step S22, the generated periodic electrical signal 41 is applied to at least one piezo-electric element 142; 242; 44; 46; 48-1; 48-2. The piezo-electric element 142; 242; 44; 46; 48-1; 48-2 is adapted to emit the ultrasound waves 2 towards the receiving portion 12; 12'; 12"; 12‴ when the periodic electrical signal 41 is applied.

In an optional step S06 following the measuring S05, a property of the fluid 1, in particular a property relating to blood or blood platelets within the fluid 1, is determined based on the measured electrical property of the fluid 1. For this, an analyzing unit may be provided within the system according to the present invention, in particular within the testing apparatus 150; 250 of the system 110; 210.

Although the exemplary embodiments of the present invention have been disclosed for illustrative purposes, those skilled in the art will appreciate that various modifications, additions and substitutions are possible, without departing from the scope of the invention as defined by the appended claims.

## Claims

1. A system comprising:
a cartridge (150, 250) and a measuring unit (26), wherein the cartridge is configured to engage with a docking portion (166) of a testing apparatus (160) and adapted to be brought into engagement with the testing apparatus (160);
the cartridge (150, 250) comprising
a receiving portion (12; 12'; 12"; 12‴) for receiving a liquid (1) to be tested, the receiving portion (12; 12'; 12"; 12‴) being formed as a double-open-ended tube structure,
at least one first electrode (21; 21'; 21"; 21‴) reaching into the receiving portion (12; 12'; 12"; 12‴),
a second electrode (22; 22'; 22"; 22‴) reaching into the receiving portion (12; 12'; 12"; 12‴),
at least one ultrasound transducer (48) positioned to emit ultrasound waves (2) towards the receiving portion (12; 12'; 12"; 12‴), and
a voltage application unit (24) adapted to apply an electrical voltage between the first electrode (21; 21'; 21"; 21‴) and the second electrode (22; 22'; 22"; 22‴);
wherein the measuring unit (26) is electrically coupled to the first electrode (21; 21'; 21";
21‴) and the second electrode (22; 22'; 22"; 22‴),
and wherein the measuring unit (26) is
configured to detect resistance or impedance of a fluid in the receiving portion (12; 12'; 12"; 12‴) to a direct or alternating current between the at least one first electrode (21; 21'; 21"; 21‴) and the second electrode (22; 22'; 22"; 22‴) through the liquid in the receiving portion (12; 12'; 12"; 12‴).

2. The system of claim 1, wherein the cartridge (150, 250) comprises an inlet (154) adapted to receive said liquid (1).

3. The system of claim 1, wherein the cartridge (150, 250) comprises a plurality of receiving portions (12; 12'; 12"; 12‴).

4. The system of claim 3, wherein each of the plurality of receiving portions (12, 12'; 12"; 12‴) comprises an own inlet (154).

5. The system of any of claims 1 to 4, wherein the cartridge (150, 250) comprises a first electrical interface (152).

6. The system of claim 5, wherein the cartridge (150, 250) comprises a third electrical interface (257).

7. The system of claim 6, wherein the first electrical interface (152) and the third electrical interface (257) are configured to mate with electrical interfaces (257, 267) integrated into the testing apparatus (160).

8. The system of claim 1, wherein the cartridge (150,250) is adapted for insertion into the docking portion (166) of the testing apparatus (160).

9. The system of claim 8, wherein the insertion is by plugging the cartridge (150, 250) into the docking portion (166) of the testing apparatus (160).

10. The system of claim 1 to 9, wherein the at least one ultrasound transducer comprises a piezo-electric element (142).

11. The system of claim 1 to 10, wherein the electrical voltage comprises alternating electrical voltage.

12. The system of any one of the claims 1 to 11, wherein the double-open-ended tube structure is a microchannel.

13. The system of any one of the claims 1 to 12, wherein the receiving portion (12; 12'; 12"; 12‴) is configured to receive the liquid (1) by exploitation of a capillary effect.

14. The system of any one of the claims 1 to 13, wherein the ultrasound transducer (48) comprises a first piezo-electric element (48-1) and a second piezo-electric element (48-2),
wherein the first piezo-electric element (48-1) and the second piezo-electric element (48-2) are arranged at diametrically opposite sides of the tube structure of the receiving portion (12; 12'; 12"; 12‴).

## Patentansprüche

1. Ein System, das Folgendes umfasst:
eine Kartusche bzw. Patrone (150, 250) und eine Messeinheit (26), wobei die Kartusche so konfiguriert ist, dass sie in einen Andockabschnitt (166) einer Testvorrichtung (160) eingreift und mit der Testvorrichtung (160) in Eingriff gebracht werden kann;
wobei di Kartusche (150, 250) Folgendes umfasst:
einen Aufnahmeabschnitt (12; 12'; 12"; 12‴) zur Aufnahme einer zu testenden Flüssigkeit (1), wobei der Aufnahmeabschnitt (12; 12'; 12"; 12‴) als eine an zwei Seiten offene Rohrstruktur ausgebildet ist,
mindestens eine erste Elektrode (21; 21'; 21"; 21‴), die in den Aufnahmeabschnitt (12; 12'; 12"; 12‴) hineinreicht,
eine zweite Elektrode (22; 22'; 22"; 22‴), die in den Aufnahmeabschnitt (12; 12'; 12"; 12‴) hineinreicht,
mindestens einen Ultraschallwandler (48), der so angeordnet ist, dass er Ultraschallwellen (2) in Richtung des Empfangsabschnitts (12; 12'; 12"; 12‴) aussendet, und
eine Spannungsanlegeeinheit (24), die dazu angepasst ist, eine elektrische Spannung zwischen der ersten Elektrode (21; 21'; 21"; 21‴) und der zweiten Elektrode (22; 22', 22"; 22‴) anzulegen;
wobei die Messeinheit (26) elektrisch mit der ersten Elektrode (21; 21'; 21"; 21‴) und der zweiten Elektrode (22; 22'; 22"; 22‴) gekoppelt ist, und
wobei die Messeinheit (26) so konfiguriert ist, dass sie den Widerstand oder die Impedanz eines Fluids im Aufnahmeabschnitt (12; 12'; 12"; 12‴) gegenüber einem Gleich- oder Wechselstrom zwischen der mindestens einen ersten Elektrode (21; 21'; 21"; 21‴) und der zweiten Elektrode (22; 22'; 22"; 22‴) durch die Flüssigkeit im Aufnahmeabschnitt (12; 12'; 12"; 12‴) hindurch erfasst.

2. Das System nach Anspruch 1, wobei die Kartusche (150, 250) einen Einlass (154) umfasst, der zur Aufnahme der genannten Flüssigkeit (1) angepasst ist.

3. Das System nach Anspruch 1, wobei die Kartusche (150, 250) eine Vielzahl von Aufnahmeabschnitten (12; 12'; 12"; 12‴) umfasst.

4. Das System nach Anspruch 3, wobei jeder der Vielzahl von Aufnahmeabschnitten (12; 12'; 12"; 12‴) einen eigenen Einlass (154) umfasst.

5. Das System nach irgendeinem der Ansprüche von 1 bis 4, wobei die Kartusche (150, 250) eine erste elektrische Schnittstelle (152) umfasst.

6. Das System nach Anspruch 5, wobei die Kartusche (150, 250) eine dritte elektrische Schnittstelle (257) umfasst.

7. Das System nach Anspruch 6, wobei die erste elektrische Schnittstelle (152) und die dritte elektrische Schnittstelle (257) so konfiguriert sind, dass sie mit elektrischen Schnittstellen (257, 267) zusammenpassen, die in die Testvorrichtung (160) integriert sind.

8. Das System nach Anspruch 1, wobei die Kartusche (150, 250) zum Einsetzen in den Andockabschnitt (166) der Testvorrichtung (160) angepasst ist.

9. Das System nach Anspruch 8, wobei das Einsetzen durch Einstecken der Kartusche (150, 250) in den Andockabschnitt (166) der Testvorrichtung (160) erfolgt.

10. Das System nach irgendeinem der Ansprüche von 1 bis 9, wobei der mindestens eine Ultraschallwandler ein piezoelektrisches Element (142) umfasst.

11. Das System nach irgendeinem der Ansprüche von 1 bis 10, wobei die elektrische Spannung eine elektrische Wechselspannung umfasst.

12. Das System nach irgendeinem der Ansprüche von 1 bis 11, wobei die an zwei Seiten offene Rohrstruktur ein Mikrokanal ist.

13. Das System nach irgendeinem der Ansprüche von 1 bis 12, wobei der Aufnahmeabschnitt (12; 12'; 12"; 12‴) so konfiguriert ist, dass er die Flüssigkeit (1) unter Ausnutzung eines Kapillareffekts aufnimmt.

14. Das System nach irgendeinem der Ansprüche von 1 bis 13, wobei der Ultraschallwandler (48) ein erstes piezoelektrisches Element (48-1) und ein zweites piezoelektrisches Element (48-2) umfasst,
wobei das erste piezoelektrische Element (48-1) und das zweite piezoelektrische Element (48-2) an diametral gegenüberliegenden Seiten der Rohrstruktur des Aufnahmeabschnitts (12; 12'; 12"; 12‴) angeordnet sind.

## Revendications

1. Un système comprenant :
une cartouche (150, 250) et une unité de mesure (26), sachant que la cartouche est configurée pour s'engager avec une portion d'accueil (166) d'un appareil de test (160) et adaptée pour être amenée en engagement avec l'appareil de test (160) ;
la cartouche (150, 250) comprenant :
une portion de réception (12 ; 12' ; 12" ; 12'") pour recevoir un liquide (1) à tester, la portion de réception (12 ; 12' ; 12" ; 12'") étant formée comme une structure tubulaire à double extrémité ouverte,
au moins une première électrode (21 ; 21' ; 21" ; 21‴) pénétrant dans la portion de réception (12 ; 12' ; 12" ; 12‴),
une deuxième électrode (22 ; 22' ; 22" ; 22‴) pénétrant dans la portion de réception (12 ; 12' ; 12" ; 12‴),
au moins un transducteur ultrasonore (48) positionné pour émettre des ondes ultrasonores (2) vers la portion de réception (12 ; 12' ; 12" ; 12'"), et
une unité d'application de tension (24) adaptée pour appliquer une tension électrique entre la première électrode (21 ; 21' ; 21" ; 21‴) et la deuxième électrode (22 ; 22', 22" ; 22‴) ;
sachant que l'unité de mesure (26) est couplée électriquement à la première électrode (21 ; 21' ; 21" ; 21‴) et à la deuxième électrode (22 ; 22' ; 22" ; 22'"), et
sachant que l'unité de mesure (26) est configurée pour détecter la résistance ou l'impédance d'un fluide dans la portion de réception (12 ; 12' ; 12" ; 12‴) à un courant continu ou alternatif entre la au moins une première électrode (21 ; 21' ; 21" ; 21‴) et la deuxième électrode (22 ; 22' ; 22" ; 22'") à travers le liquide dans la portion de réception (12 ; 12' ; 12" ; 12‴).

2. Le système d'après la revendication 1, sachant que la cartouche (150, 250) comprend une entrée (154) adaptée pour recevoir ledit liquide (1).

3. Le système d'après la revendication 1, sachant que la cartouche (150, 250) comprend une pluralité de portions de réception (12 ; 12' ; 12" ; 12‴).

4. Le système d'après la revendication 3, sachant que chacune de la pluralité de portions de réception (12 ; 12' ; 12" ; 12‴) comprend une propre entrée (154).

5. Le système d'après l'une quelconque des revendications de 1 à 4, sachant que la cartouche (150, 250) comprend une première interface électrique (152).

6. Le système d'après la revendication 5, sachant que la cartouche (150, 250) comprend une troisième interface électrique (257).

7. Le système d'après la revendication 6, sachant que la première interface électrique (152) et la troisième interface électrique (257) sont configurées pour s'accoupler avec des interfaces électriques (257, 267) intégrées dans l'appareil de test (160).

8. Le système d'après la revendication 1, sachant que la cartouche (150, 250) est adaptée pour être insérée dans la portion d'accueil (166) de l'appareil de test (160).

9. Le système d'après la revendication 8, sachant que l'insertion se fait en enfichant la cartouche (150, 250) dans la portion d'accueil (166) de l'appareil de test (160).

10. Le système d'après la revendication 1 à 9, sachant que le au moins un transducteur ultrasonore comprend un élément piézoélectrique (142).

11. Le système d'après la revendication 1 à 10, sachant que la tension électrique comprend une tension électrique alternative.

12. Le système d'après l'une quelconque des revendications de 1 à 11, sachant que la structure tubulaire à double extrémité ouverte est un microcanal.

13. Le système d'après l'une quelconque des revendications de 1 à 12, sachant que la portion de réception (12 ; 12' ; 12" ; 12‴) est configurée pour recevoir le liquide (1) par exploitation d'un effet capillaire.

14. Le système d'après l'une quelconque des revendications de 1 à 13, sachant que le transducteur ultrasonore (48) comprend un premier élément piézo-électrique (48-1) et un deuxième élément piézo-électrique (48-2),
sachant que le premier élément piézo-électrique (48-1) et le deuxième élément piézo-électrique (48-2) sont disposés sur des côtés diamétralement opposés de la structure tubulaire de la portion de réception (12 ; 12' ; 12" ; 12'").
